(19) Europäisches Patentamt
European Patent Office
Office européen des brevets

(11) Veröffentlichungsnummer: **0 046 731**
**B1**

(12) # EUROPÄISCHE PATENTSCHRIFT

(45) Veröffentlichungstag der Patentschrift: **03.10.84**

(51) Int. Cl.³: **C 07 C 41/16**, C 07 C 43/188, C 07 C 43/14, C 08 F 16/32

(21) Anmeldenummer: **81810338.4**

(22) Anmeldetag: **19.08.81**

(54) **Verfahren zur Herstellung von Di- und Polyallyläthern.**

(30) Priorität: **25.08.80 CH 6386/80**

(43) Veröffentlichungstag der Anmeldung: **03.03.82 Patentblatt 82/9**

(45) Bekanntmachung des Hinweises auf die Patenterteilung: **03.10.84 Patentblatt 84/40**

(84) Benannte Vertragsstaaten: **CH DE FR GB LI NL**

(56) Entgegenhaltungen:
**DE - B - 1 102 728**
**US - A - 3 840 605**

**Angew. Chemie 89 (1977) 7, Seiten 521, 522**

(73) Patentinhaber: **CIBA-GEIGY AG, Postfach, CH-4002 Basel (CH)**

(72) Erfinder: **Lohse, Friedrich, Dr. Prof., Buchenstrasse 23, CH-4104 Oberwil (CH)**
Erfinder: **Monnier, Charles E., Dr., Hirzbodenweg 17/1, CH-4052 Basel (CH)**

**EP 0 046 731 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

ACTORUM AG

## Beschreibung

Die vorliegende Erfindung betrifft ein Verfahren zur Herstellung von Allyläthern durch Umsetzung von einen aliphatischen, araliphatischen, cycloaliphatischen, cycloaliphatisch-aliphatischen oder heterocyclisch-aliphatischen Rest enthaltenden Di- und Polyhydroxyverbindungen mit Allylchlorid oder -bromid nach der Phasentransferkatalyse.

Allyläther aliphatischer Alkohole lassen sich bekanntlich in einfacher Weise nach der Äther-Synthese von Williamson aus Alkalialkoholaten und Allylhalogeniden herstellen. Schwierigkeiten bereitet dagegen oft die Herstellung von Alkoholaten höhermolekularer Alkohole, insbesondere von sekundären und tertiären Alkoholen, da diese nur schwer mit zum Beispiel Natrium reagieren.

In US-A-3 840 605 wird ein Verfahren zur Herstellung von Äthern durch Umsetzung von aliphatischen Alkoholen mit Alkylhalogeniden in Gegenwart von organischen quaternären Salzen als Katalysatoren beschrieben, wobei alkalifrei gearbeitet wird und Ausbeuten erhalten werden, die noch nicht zufriedenstellend sind.

Aus «Tetrahedron Letters», No. 38 (1975), Seite 3251–3254, geht hervor, dass man mit einer verbesserten Äther-Synthese nach Williamson, durch Anwendung der Phasentransferkatalyse, asymmetrische Äther in hohen Ausbeuten erhält. Aus dieser Publikation geht indessen nirgends hervor, dass bei Verwendung von mehrwertigen Alkoholen, insbesondere von mehrwertigen sekundären oder tertiären Alkoholen, die Phasentransferkatalyse zu einem hohen Allylierungsgrad aller Hydroxylgruppen des mehrwertigen Alkohols führt.

Auch in «Angewandte Chemie», 89 (1977), Seite 521 bis 533, worin über den Mechanismus und über Fortschritte der Phasentransferkatalyse berichtet wird und unter anderem Kronenäther als geeignete Katalysatoren für die Phasentransferkatalyse beschrieben werden, befindet sich kein Hinweis über die Herstellung von Allyläthern mehrwertiger Alkohole.

Es wurde nun gefunden, dass durch Umsetzung von mehrwertigen Alkoholen mit Allylchlorid oder -bromid nach der Phasentransferkatalyse Umsetzungsprodukte mit einem hohen Allylierungsgrad und in hohen Ausbeuten erhalten werden.

Gegenstand der vorliegenden Erfindung ist somit ein Verfahren zur Herstellung von Allyläthern der Formel I

$$R -(O-CH_2-CH=CH_2)_n \qquad (I),$$

worin R einen n-wertigen aliphatischen, araliphatischen, cycloaliphatischen, cycloaliphatisch-aliphatischen oder heterocyclisch-aliphatischen Rest und n eine Zahl von mindestens 2 bedeuten, durch Umsetzung von Hydroxyverbindungen der Formel II

$$R -(OH)_n \qquad (II),$$

worin R und n die gleiche Bedeutung wie in Formel I haben und die OH-Gruppen an primäre, sekundäre oder tertiäre aliphatische C-Atome gebunden sind, mit Allylchlorid oder -bromid in Gegenwart eines Katalysators und im alkalischen Medium, dadurch gekennzeichnet, dass man die Umsetzung mittels der Phasentransferkatalyse durchführt, wobei man pro 1 Hydroxyläquivalent der Verbindung der Formel II 0,8 bis 5 Mole Allylchlorid oder -bromid, 1 bis 6 Mole wässrige oder feste Natronlauge und 2 bis 20 Mol-% eines quaternären Ammoniumsalzes, einer quaternären Ammoniumbase oder eines Kronenäthers als Phasenumwandlungskatalysator einsetzt und die Umsetzung im Temperaturbereich von 20 bis 100 °C durchführt.

Vorzugsweise setzt man Hydroxyverbindungen der Formel II ein, worin R einen n-wertigen aliphatischen, araliphatischen, cycloaliphatischen, cycloaliphatisch-aliphatischen oder heterocyclisch-aliphatischen Rest mit bis zu 120 C-Atomen und n eine Zahl von 2 bis 6 bedeuten.

Insbesondere verwendet man bei dem erfindungsgemässen Verfahren als Hydroxyverbindung der Formel II primäre oder sekundäre Alkohole.

Die Ausgangsstoffe für die Herstellung der Allyläther der Formel I stellen bekannte Verbindungen dar und werden vorzugsweise in solchen Mengen eingesetzt, dass im Reaktionsgemisch pro 1 Hydroxyläquivalent der Verbindung der Formel II 0,9 bis 2,5 Mole Allylchlorid oder -bromid enthalten sind. Insbesondere setzt man die Ausgangsstoffe in äquivalenten Mengen ein.

Die Umsetzungsreaktion kann sowohl in Gegenwart von festem NaOH als auch von wässrigen NaOH-Lösungen durchgeführt werden. In der Regel verwendet man 20 bis 99%-ige wässrige NaOH-Lösungen, vorzugsweise 30 bis 80%-ige, und insbesondere eine 50%-ige wässrige NaOH-Lösung.

Die Umsetzungsreaktion wird ferner vorzugsweise im Temperaturbereich von 25 bis 80 °C, insbesondere zwischen 50 und 75 °C durchgeführt.

Als geeignete Dihydroxyverbindungen der Formel II seien beispielsweise genannt: Aliphatische Diole, wie Äthylenglykol, Propan-1,3-diol, Propan-1,2-diol, Neopentylglykol, Butan-1,4-diol, Butan-1,3-diol, Hexan-1,6-diol, 2,2-Diäthylpropan-1,3-diol, 2-Methyl-2-propyl-propan-1,3-diol, 2,2,4- bzw. 2,4,4-Trimethyl-hexan-1,6-diol, 2-Methyl-2-äthyl-propan-1,3-diol, Dodecan-1,12-diol oder Hydroxypivalinsäureneopentylglykolester, ungesättigte Diole, wie Buten-1,4-diol oder Butin-1,4-diol, Polyalkylenglykole, wie Diäthylenglykol, Triäthylenglykol oder Dipropylenglykol, als araliphatische Diole 1,4-Bis-(hydroxymethyl)-benzol, 1,4-Bis-(hydroxyäthyläther) von Bisphenol A, Bisphenol F oder Hydrochinon, ferner cycloaliphatische und cycloaliphatisch-aliphatische Diole, wie Cyclohexan-1,2-diol, Cyclohexan-1,3-diol, Cyclohexan-1,4-diol, hydriertes Bisphenol A oder Bisphenol F, 1,1-, 1,2-, 1,3- und 1,4-Bis-(hydroxymethyl)-cyclohexan und die entsprechenden ungesättigten Cyclohexenderivate, wie 1,1-Bis-(hydroxymethyl)-cyclohexen, 1,4-Bis-(hydroxymethyl)-cyclohexen sowie die heterocyclisch-aliphatischen Diole, wie sie durch Addition von 2 oder mehr Molen Alkylenoxid, wie Äthylenoxid, Propylenoxid, Butylenoxid, Styroloxid oder Cyclohexenoxid, insbesondere Äthylenoxid, an 1 Mol einer einkernigen oder mehrkernigen N-heterocyclischen Verbindung, wie Hydantoin und seine Derivate, Dihydrouracil und seine Derivate, Barbitursäure und ihre Derivate, Benzimidazolon sowie Tetrahydrobenzimidazolon und ihre Derivate, Bishydantoin sowie Bis-dihydrouracil und ihre Derivate, erhalten werden. Solche Verbindungen sind beispielsweise
1,3-Di-(β-hydroxyäthyl)-5,5-dimethylhydantoin,
1,3-Di-(β-hydroxyäthyl)-5-isopropylhydantoin,
1,3-Di-(β-hydroxyäthyl)-benzimidazolon,
1,3-Di-(β-hydroxyäthyl)-tetrahydrobenzimidazolon und
1,1'-Methylen-bis-(3-β-hydroxyäthyl-5,5-dimethylhydantoin), und die langkettigen, einen heterocyclischen Rest enthaltende Diole sind beispielsweise in den DE-Offenlegungsschriften 1 954 503 und 2 003 016 beschrieben.

Als Tri-, Tetra-, Penta- und Hexahydroxyverbindungen der Formel II seien beispielsweise genannt: 1,1,1-Tri-(hydroxymethyl)-äthan, 1,1,1-Tri-(hydroxymethyl)-propan, Tri-(hydroxymethyl)-nitromethan, Glycerin, Hexan-1,2,6-triol, Butan-1,2,4-triol sowie die durch Anlagerung von Alkylenoxid, insbesondere 1–3 Mol Alkylenoxid, wie Äthylenoxid, Propylenoxid, Styroloxid oder Cyclohexenoxid, insbesondere Äthylenoxid, an diese Triole erhaltenen Additionsprodukte, N-heterocyclische Trihydroxyverbindungen, wie 1,3,5-Tris-(hydroxyäthyl)-isocyanurat, ferner Pentaerythrit, Erythrit, Xylit, Arabit, Mannit, Sorbit und Dipentaerythrit.

Als Polyhydroxyverbindungen können beim erfindungsgemässen Verfahren auch die Polyvinylalkohole eingesetzt werden, insbesondere solche mit einem durchschnittlichen Molekulargewicht bis zu etwa 15 000.

Als Phasenumwandlungskatalysatoren können bei dem erfindungsgemässen Verfahren quaternäre Ammoniumsalze, wie beispielsweise Tetramethylammoniumchlorid, Tetraäthylammoniumchlorid, Benzyltrimethylammoniumchlorid, Benzyltrimethylammoniumacetat, Methyltriäthylammoniumchlorid, Tetrabutylammoniumchlorid oder Tetrabutylammoniumsulfat, quaternäre Ammoniumbasen, wie Benzyltrimethylammoniumhydroxid, sowie die Kronenäther, beispielsweise 12-Kronen-4-äther (1,4,7,10-Tetraoxacyclododecan), 15-Kronen-5-äther (1,4,7,10,13-Pentaoxacyclopentadecan), 18-Kronen-6-äther, Dibenzo-18-Kronen-6-äther, Dibenzo-24-Kronen-8-äther, Dibenzo-1,4-dioxa-8,12-diazacyclopentadeca-5,14-dien, Dicyclohexano-18-Kronen-6-äther oder Dicyclohexano-24-Kronen-8-äther, eingesetzt werden.

Die vorgenannten Kronenäther sind bekannte Verbindungen und im Handel erhältlich.

Die nach dem erfindungsgemässen Verfahren herstellbaren Verbindungen sind grösstenteils bekannt. Gegenstand vorliegender Erfindung sind auch Allyläther der Formel I, worin R einen Rest der Formeln

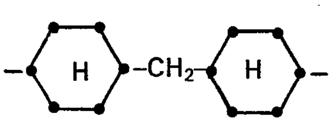

oder

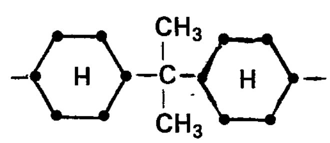

bedeutet und n für die Zahl 2 steht, sowie das Isomerengemisch aus o,o-, o,p- und p,p-Perhydrobisphenol-F-diallyläther.

Die nach dem erfindungsgemässen Verfahren erhaltenen Allyläther stellen wertvolle Monomere dar, die allein oder in Mischung mit anderen polymerisierbaren Monomeren, wie zum Beispiel Diallylphthalat, Triallylcyanurat oder Styrol in vernetzte Polymeren überführt werden können. Die nach dem erfindungsgemässen Verfahren erhaltenen Allyläther sind auch wertvolle Ausgangsprodukte zur Herstellung von Epoxidverbindungen nach dem Epoxidationsverfahren, wobei im Vergleich zur Herstellung von Epoxidharzen mittels Epoxidhalogenhydrin, halogenfreie Epoxidharze erhalten werden.

Allgemeine Vorschrift zur Herstellung von Allyläthern

In einem 0,3 l Stahlautoklaven mit Magnetrührer werden 0,1 Mol Di- oder Polyhydroxyverbindung der Formel II, 0,1–0,6 Mol 50%ige wässrige NaOH, 0,25 Mol Allylchlorid und 20 Mol-% Tetrabutylammoniumhydrogensulfat, bezogen auf 1 Hydroxyläquivalent Alkohol, in 60 ml eines inerten, organischen Lösungsmittels, zum Beispiel Toluol oder Xylol, vorgelegt. Das Gemisch wird auf 50–55 °C erwärmt und 5 Stunden bei dieser Temperatur gerührt (etwa 1000 Umdrehungen pro Minute). Anschliessend wird das Reaktionsgemisch noch bei 70–75 °C während 15 Stunden gerührt. Das Reaktionsgemisch wird 3 mal mit 100 ml Wasser gewaschen, die organische Phase über Natriumsulfat getrocknet und eingeengt. Das Rohprodukt wird unter vermindertem Druck bei etwa 0,13 mbar destilliert.

Bei Herstellung der Allyläther nach obiger Arbeitsvorschrift kann auf die Verwendung eines organischen Lösungsmittels auch verzichtet werden.

Beispiel 1

In einen 1 Liter Stahlautoklaven werden 64,0 g (0,27 Mol) Perhydrobisphenol-A (hydriertes Bisphenol A), 243,3 g (2,7 Mol) 50%ige wässrige NaOH, 204,0 g (2,7 Mol) Allylchlorid und 36,3 g

(0,11 Mol) Tetrabutylammoniumhydrogensulfat in 160 ml Xylol vorgelegt. Das Gemisch wird unter starkem Rühren (etwa 1000 Umdrehungen pro Minute) während 5 Stunden auf 55–60 °C, und anschliessend während 15 Stunden auf 70 °C gehalten. Nach Beenden der Reaktion wird das Reaktionsgemisch 3 mal mit etwa 200 ml Wasser gewaschen, die organische Phase über Natriumsulfat getrocknet und eingeengt. Es resultieren 81,13 g (93% der Theorie) eines gelblichen, niedrig viskosen Harzes. Nach Destillation des Rohproduktes bei 159–160 °C/0,09 mbar werden 73,11 g (86% der Theorie) farbloses Öl erhalten (Gehalt: 81,1 Gew.-% Diallyläther, 15,4 Gew.-% Monoallyläther und ca. 3,5 Gew.-% Edukt). Der reine Perhydrobisphenol-A-diallyläther siedet bei 159–160 °C/0,11 mbar und stellt ein farbloses Öl dar.

Beispiel 2

In einem 0,3 Liter Stahlautoklaven mit Magnetrührer werden 6,2 g (0,1 Mol) Äthylenglykol, 40 g (0,5 Mol) 50%ige wässrige NaOH, 20 Mol % Tetrabutylammoniumhydrogensulfat pro 1 Hydroxyläquivalent des Glykols und 38 g (0,5 Mol) Allylchlorid in 60 ml Toluol vorgelegt. Das Gemisch wird unter Rühren auf 50–55 °C während 5 Stunden erwärmt, anschliessend wird noch während 15 Stunden bei 70–75 °C weiter gerührt. Das Reaktionsprodukt wird 3 mal mit 100 ml Wasser gewaschen, die organische Phase über Natriumsulfat getrocknet und eingeengt. Es werden 9,2 g eines farblosen Öls erhalten, welches bei 110 °C/ 33 mbar destilliert wird. Ausbeute: 8,7 g (61% der Theorie) Äthylenglykoldiallyläther (Reinheit >95%).

Beispiel 3

Vorgelegt werden 11,8 g (0,1 Mol) Hexan-1,6-diol, 40 g (0,5 Mol) 50%ige wässrige NaOH, 20 Mol% Tetrabutylammoniumhydrogensulfat pro 1 Hydroxyläquivalent des Diols und 38 g (0,5 Mol) Allylchlorid in 60 ml Toluol. Das Gemisch wird während 15 Stunden bei 80 °C erwärmt. Nach Aufarbeiten werden 17,28 g (87% der Theorie) farbloses Öl erhalten, welches nach Destillation bei 90 °C/0,04 mbar 16,84 g (85% der Theorie) farbloses Hexan-1,6-diallyläther liefert (Reinheit >95%).

Beispiel 4

Vorgelegt werden 11,8 g (0,1 Mol) Cyclohexan-1,4-diol, 40 g (0,5 Mol) 50%ige wässrige NaOH, 20 Mol% Tetrabutylammoniumhydrogensulfat pro 1 Hydroxyläquivalent des Diols und 38 g (0,5 Mol) Allylchlorid. Gemäss Beispiel 2 werden nach 15 stündiger Reaktionszeit bei 80 °C 14,11 g (72%) farblosen Cyclohexan-1,4-diallyläther erhalten, welcher bei 110–120 °C/0,07 mbar destilliert wird und 13,45 g (69% der Theorie) Allyläther, bestehend aus 94 Gew.-% Cyclohexan-1,4-diallyläther und 6 Gew.-% p,p'-Cyclohexanolallyläther, liefert.

Beispiel 5

Unter den in Beispiel 2 beschriebenen Bedingungen werden aus 6,8 g (0,05 Mol) Pentaerythrit und 38 g (0,5 Mol) Allylchlorid 14,7 g (99% der Theorie) Pentaerythrittetraallyläther erhalten, welcher nach Destillation bei 160 °C/0,05 mbar 13,75 g (93% der Theorie) Pentaerythrittetraallyläther liefert (Reinheit 99%).

Beispiel 6

Nach Beispiel 2 werden aus 13,4 g (0,1 Mol) Trimethylolpropan und 57 g (0,57 Mol) Allylchlorid 24,9 g (98% der Theorie) Trimethylolpropanallyläther erhalten. Nach Destillation bei 120 °C/0,08 mbar werden 23,55 g (93% der Theorie) Trimethylolpropanallyläther, bestehend aus 79 Gew.-% Trimethylolpropantriallyläther und 21 Gew.-% Trimethylolpropandiallyläther, erhalten.

Beispiel 7

Wie in Beispiel 2 beschrieben, werden 10,4 g (0,1 Mol) Neopentylglykol mit 38 g (0,5 Mol) Allylchlorid umgesetzt, wobei 14,96 g (81% der Theorie) Neopentylglykolallyläther erhalten werden, welcher bei 130 °C/15 mbar destilliert wird und 14,4 g (78% der Theorie) Neopentylallyläther, bestehend aus 91 Gew.-% Neopentyldiallyläther und 9 Gew.-% Neopentylmonoallyläther, liefert.

Beispiel 8

a) In einem 6,3 Liter-Stahlautoklaven werden 482,5 g (2,28 Mol) eines Isomerengemisches von Perhydrobisphenol-F (o,o-/o,p-/p,p- ca. 10%/50%/30%), 480 g (12 Mol) Natriumhydroxidplättchen, 918 g (12 Mol) Allylchlorid, 1200 ml Toluol und 82 g (5 Mol% pro 1 Hydroxyläquivalent des Perhydrobisphenols F) Tetrabutylammoniumhydrogensulfat vorgelegt. Das Gemisch wird unter starkem Rühren während 5 Stunden auf 50 °C erwärmt, anschliessend noch während 15 Stunden bei 80 °C nachreagieren gelassen. Nach Beendigen der Reaktion wird das Reaktionsgemisch 3 mal mit etwa 1 Liter Wasser gewaschen und am Vakuum eingeengt.

Es resultieren 558,3 g (84% der Theorie) leicht gelbliches Perhydrobisphenol-F-diallyläther. Destillation bei 130–135%C/0,13 mbar liefert 355,6 g reinen, farblosen Perhydrobisphenol-F-diallyläther.

b) In einem 4,5 Liter-Sulfierkolben mit Rührer, Thermometer, Kühler und Tropftrichter werden 643,4 g (2,2 Mol) des oben erhaltenen Perhydrobisphenol-F-diallyläthers und 241,5 g (5,25 Mol) Ameisensäure in 2500 ml Chlorbenzol vorgelegt. Innerhalb etwa einer Stunde werden zum Gemisch bei 45–50 °C 510 g (10,5 Mol) 70%-ige Wasserssoffperoxidlösung hinzugetropft. Man rührt noch weitere 6 Stunden bei 50–55 °C. Nach Beendigen der Reaktion wird die organische Phase mit Natriumbisulfit von Peroxid freigewaschen und danach mit Wasser gewaschen, über Natriumsulfat getrocknet und am Vakuum eingeengt. Es resultieren 661,8 g (93% der Theorie)

leicht viskoser, farbloser Perhydrobisphenol-F-diglycidyläther mit einem Epoxidgehalt von 4,91 Äquivalenten/kg (Theorie 6,16 Äquivalente/kg). Das Produkt zeigt bei 25 °C eine Viskosität von 275 mPa·s.

Beispiel 9

Anstelle von Tetrabutylammoniumhydrogensulfat gemäss Beispiel 1 wird 18-Kronen-6-äther als Phasentransferkatalysator verwendet.

In einem 200 ml Sulfierkolben mit Rührer, Kühler, Thermometer und Tropftrichter werden 12,0 g (0,037 Mol) Perhydrobisphenol-A, 20,0 g pulverisierte Natronlauge, 0,53 g 18-Kronen-6-äther (2 Mol% pro 1 Hydroxyläquivalent) und 60 ml Toluol auf 60 °C erwärmt. Innerhalb einer Stunde werden 18,15 g (0,15 Mol) Allylbromid bei 70 °C hinzugetropft. Das Reaktionsgemisch wird während 20 Stunden bei 70 °C gerührt, anschliessend mit 200 ml Wasser versetzt und die wässrige Phase abgetrennt. Die organische Phase wird mit 200 ml 5%iger Salzsäure gewaschen, über $Na_2SO_4$ getrocknet und eingeengt. Es resultieren 13,51 g (84,3% der Theorie) eines leicht gelblichen, viskosen Öls der Zusammensetzung: Diallyläther 53 Gew.-% Monoallyläther: 31 Gew.-% und nicht umgesetztes Perhydrobisphenol-A 11 Gew.-%.

Beispiel 10

In einem 1 Liter-Stahlautoklaven werden 96 g (0,4 Mol) Perhydrobisphenol-A (hydriertes Bisphenol-A), 80 g (2,0 Mol) Natriumhydroxid Plättchen, 153 g (2,0 Mol) Allylchlorid und 13,6 g n-Dodecyldimethylbenzylammoniumchlorid und 200 ml Toluol vorgelegt. Das Gemisch wird unter starkem Rühren (etwa 1000 Umdrehungen pro Minute) während 5 Stunden auf 55–60 °C und anschliessend während 15 Stunden auf 80 °C gehalten. Nach Beenden der Reaktion wird das Reaktionsgemisch 3 mal mit ca. 500 ml Wasser gewaschen, die organische Phase über Natriumsulfat getrocknet, filtriert und am Vakuum eingeengt. Es resultieren 123,5 g (96,34% der Theorie) Perhydrobisphenol-A-diallyläther (Reinheit >95%).

Beispiel 11

In einem 750 ml Sulfierkolben mit Rührer, Kühler, Thermometer und Tropftrichter werden 43,2 g (0,3 Mol) 1,4-Bis-(hydroxymethyl)-cyclohexan, 120 g (3,0 Mol) pulverisierte Natronlauge, 20,4 g Tetrabutylammoniumhydrogensulfat (10 Mol% pro 1 Hydroxyläquivalent) und 180 ml Toluol vorgelegt. Das Gemisch wird auf 70 °C erwärmt und innerhalb von etwa einer Stunde werden 181,5 g (1,5 Mol) Allylbromid hinzugetropft. Es wird während 12 Stunden bei 80 °C weitergerührt. Nach Beenden der Reaktion wird das Reaktionsgemisch mit 200 ml 5%iger Salzsäurelösung und 700 ml Wasser gewaschen. Die organische Phase wird über $Na_2SO_4$ getrocknet und eingeengt. Es resultieren 61,1 g (90,64% der Theorie) leicht viskoser, gaschromatographisch einheitlicher 1,4-Bis-(2-hydroxymethyl)-cyclohexandiallyläther.

Beispiel 12

In einem 750 ml Sulfierkolben mit Rührer, Thermometer, Kühler und Tropftrichter werden 41,4 g (0,3 Mol) Xylylenglykol, 120 g (3,0 Mol) Natronlauge, 20,4 g Tetrabutylammoniumhydrogensulfat (10 Mol% pro 1 Hydroxyläquivalent) und 180 ml Toluol vorgelegt. Das Gemisch wird auf 70 °C erwärmt. Anschliessend werden 181,5 g (1,5 Mol) Allylbromid innerhalb einer Stunde zugetropft. Das Reaktionsgemisch wird während weiteren 12 Stunden bei 82–84 °C gerührt. Nach Beenden der Reaktion wird das Reaktionsgemisch nacheinander mit 500 ml Wasser, 300 ml 5%iger Salzsäurelösung und 300 ml Wasser gewaschen. Die organische Phase wird über Natriumsulfat getrocknet und eingeengt, wobei 64,43 g (98% der Theorie) eines leicht gelblichen, gaschromatographisch einheitlichen Öls, Xylylenglykoldiallyläther, erhalten werden.

Beispiel 13

Vorgelegt werden in einem 350 ml Sulfierkolben mit Thermometer, Rührer und Tropftrichter 7,6 g (0,05 Mol) Xylit, 100 g 50%ige Natronlauge und 5 Mol% pro 1 Hydroxyläquivalent des Xylits Tetrabutylammoniumhydrogensulfat in 60 ml Toluol. Das Gemisch wird auf 70–75 °C erwärmt und 78 g Allylbromid (0,625 Mol) unter starkem Rühren hinzugetropft. Nach 16 stündiger Reaktionszeit wird die wässerige Phase des Reaktionsgemisches abgetrennt, die organische Phase 3 mal mit 70 ml Wasser gewaschen, über Natriumsulfat getrocknet und eingeengt. Es resultieren 11,81 g eines leicht bräunlichen Öls, welches bei 180–200 °C/0,03 mbar destilliert wird, wobei 9,40 g (53% der Theorie) als farbloses Öl erhalten werden (Reinheit >98%).

Beispiel 14

Wie im vorgehenden Beispiel beschrieben, werden 4,55 g (0,025 Mol) D-Sorbit, 5,2 g Tetrabutylammoniumhydrogensulfat (10 Mol% pro 1 Hydroxyläquivalent) und 100 ml 50%ige Natronlauge in 30 ml Toluol vorgelegt. Das Gemisch wird auf etwa 75 °C erwärmt und 90,05 g (0,75 Mol) Allylbromid werden unter starkem Rühren innerhalb von etwa 30 Minuten hinzugetropft. Das Reaktionsgemisch wird während 16 Stunden bei 75 °C weitergerührt. Nach Beenden der Reaktion wird die wässerige Phase abgetrennt, die organische Phase wird 3 mal mit 70 ml Wasser gewaschen, über Natriumsulfat getrocknet und eingeengt. Es resultieren 9,70 g (92% der Theorie) eines gelblichen Öls, welches nach Destillation bei 180–200 °C/0,01 mbar 8,45 g (86,17% der Theorie) Sorbithexaallyläther liefert (Reinheit >98%).

Beispiel 15

Vorgelegt werden 4,55 g (0,025 Mol) Mannit, 100 ml 50%ige Natronlauge und 10 Mol% pro 1 Hydroxyläquivalent des Mannits Tetrabutylammoniumhydrogensulfat in 30 ml Toluol. Das Gemisch wird auf 75–80 °C erwärmt und innerhalb 30 Minuten werden 90,05 g (0,75 Mol) Allylbromid unter starkem Rühren hinzugetropft. Das Reaktionsgemisch wird während 14 Stunden bei dieser Temperatur gerührt. Nach Beenden der Reaktion wird die wässerige Phase abgetrennt und die organische Phase 3 mal mit etwa 70 ml Wasser gewaschen, über Natriumsulfat getrocknet und eingeengt. Nach Destillation im Kugelrohr bei 50–170 °C/0,04 mbar werden 8,75 g (83% der Theorie) Mannithexaallyläther als farblose Flüssigkeit erhalten (Reinheit >98%).

Beispiel 16

Vorgelegt werden in einem 0,3 Liter Stahlautoklaven 8,7 g (0,2 OH-Äquivalente) Polyvinylalkohol (mittleres Molekulargewicht 2000), 80 g (1 Mol) 50%ige Natronlauge, 39 g (0,5 Mol) Allylchlorid, 6,8 g Tetrabutylammoniumhydrogensulfat (10 Mol% pro 1 Hydroxyläquivalent des Alkohols) und 60 ml Toluol. Das Gemisch wird unter starkem Rühren (etwa 1000 Umdrehungen pro Minute) während 24 Stunden bei 75–85 °C gerührt. Nach Beenden der Reaktion wird das Reaktionsgemisch 4 mal mit je 100 ml Wasser gewaschen, über Natriumsulfat getrocknet, filtriert und eingeengt. Es resultieren 13,4 g (79,7% der Theorie) eines klaren, hochviskosen Harzes, welches 9,3 Doppelbindungen/kg (78% der Theorie) enthält.

Beispiel 17

In einem 350 ml Sulfierkolben werden 8,7 g (0,2 OH-Äquivalente) Polyvinylalkohol (mittleres Molekulargewicht 14000), 80 g (1,0 Mol) 50%ige Natronlauge, 7,8 g Tetrabutylammoniumhydrogensulfat (10 Mol% pro 1 Hydroxyläquivalent des Alkohols) und 60 ml Toluol vorgelegt. Das Reaktionsgemisch wird auf 65–75 °C erwärmt und unter starkem Rühren werden 60,5 g (0,5 Mol) Allylbromid innerhalb etwa 60 Minuten hinzugetropft. Das Reaktionsgemisch wird während 24 Stunden weitergerührt. Nach Beenden der Reaktion wird die organische Phase 3 mal mit je 100 ml Wasser gewaschen, über Natriumsulfat getrocknet, filtriert und am Vakuum eingeengt. Es werden 10,21 g eines braunen Harzes (61,14% der Theorie) erhalten, welches 7,4 Doppelbindungen/kg (62% der Theorie) enthält.

**Patentansprüche**

1. Verfahren zur Herstellung von Allyläthern der Formel I

$$R{-}(O{-}CH_2{-}CH{=}CH_2)_n \qquad (I),$$

worin R einen n-wertigen aliphatischen, araliphatischen, cycloaliphatischen, cycloaliphatisch-aliphatischen oder heterocyclisch-aliphatischen Rest und n eine Zahl von mindestens 2 bedeuten, durch Umsetzung von Hydroxyverbindungen der Formel II

$$R{-}(OH)_n \qquad (II),$$

worin R und n die gleiche Bedeutung wie in Formel I haben und die OH-Gruppen an primäre, sekundäre oder tertiäre aliphatische C-Atome gebunden sind, mit Allylchlorid oder -bromid in Gegenwart eines Katalysators und im alkalischen Medium, dadurch gekennzeichnet, dass man die Umsetzung mittels der Phasentransferkatalyse durchführt, wobei man pro 1 Hydroxyläquivalent der Verbindung der Formel II 0,8 bis 5 Mole Allylchlorid oder -bromid, 1 bis 6 Mole wässrige oder feste Natronlauge und 2 bis 20 Mol-% eines quaternären Ammoniumsalzes, einer quaternären Ammoniumbase oder eines Kronenäthers als Phasenumwandlungskatalysator einsetzt und die Umsetzung im Temperaturbereich von 20 bis 100 °C durchführt.

2. Verfahren gemäss Anspruch 1, dadurch gekennzeichnet, dass man Hydroxyverbindungen der Formel II einsetzt, worin R einen n-wertigen aliphatischen, araliphatischen, cycloaliphatischen, cycloaliphatisch-aliphatischen oder heterocyclisch-aliphatischen Rest mit bis zu 120 C-Atomen und n eine Zahl von 2 bis 6 bedeuten.

3. Verfahren gemäss Anspruch 1, dadurch gekennzeichnet, dass man pro 1 Hydroxyläquivalent der Verbindung der Formel II 0,9 bis 2,5 Mole Allylchlorid oder -bromid verwendet.

4. Verfahren gemäss Anspruch 1, dadurch gekennzeichnet, dass man als Verbindung der Formel II primäre oder sekundäre Alkohole verwendet.

5. Verfahren gemäss Anspruch 1, dadurch gekennzeichnet, dass man die Umsetzung in Gegenwart einer 20 bis 99%igen wässrigen NaOH-Lösung durchführt.

6. Verfahren gemäss Anspruch 1, dadurch gekennzeichnet, dass man die Umsetzung in Gegenwart einer 30 bis 80%igen wässrigen NaOH-Lösung durchführt.

7. Verfahren gemäss Anspruch 1, dadurch gekennzeichnet, dass man die Umsetzung im Temperaturbereich von 25 bis 80 °C durchführt.

8. Verfahren gemäss Anspruch 1, dadurch gekennzeichnet, dass man die Umsetzung im Temperaturbereich von 50 bis 75 °C durchführt.

9. Allyläther gemäss Formel I des Anspruchs 1, dadurch gekennzeichnet, dass R einen Rest der Formeln

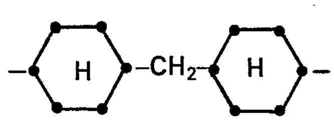

oder

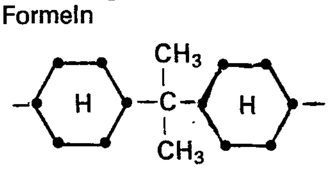

bedeutet und n für die Zahl 2 steht.

10. Isomerengemisch aus o,o-, o,p- und p,p-Perhydrobisphenol-F-diallyläther als Verbindungen der Formel I gemäss Anspruch 1.

**Revendications**

1. Procédé pour préparer des éthers allyliques répondant à la formule I:

$R-(O-CH_2-CH=CH_2)_n$ (I)

dans laquelle R représente un radical aliphatique, araliphatique, cycloaliphatique, cycloaliphatique-aliphatique ou hétérocyclique-aliphatique dont la valence est égale à n, et n désigne un nombre au moins égal à 2, par réaction de composés hydroxyliques répondant à la formule II:

$R-(OH)_n$ (II)

dans laquelle R et n ont les mêmes significations que dans la formule I et dans laquelle les radicaux –OH sont portés par des atomes de carbone aliphatiques primaires, secondaires ou tertiaires, avec le chlorure d'allyle ou le bromure d'allyle, en présence d'un catalyseur et en milieu alcalin, procédé caractérisé en ce qu'on effectue la réaction au moyen de la catalyse à transfert de phase, en utilisant, par équivalent d'hydroxy du composé de formule II, de 0,8 à 5 mol de chlorure ou de bromure d'allyle, de 1 à 6 mol d'hydroxyde de sodium solide ou sous la forme d'une solution aqueuse et, comme catalyseur à transfert de phase, de 2 à 20 mol % d'un sel d'ammonium quaternaire, d'une base d'ammonium quaternaire ou d'un éther en couronne, et on opére dans l'intervalle de température allant de 20 à 100 °C.

2. Procédé selon la revendication 1 caractérisé en ce qu'on utilise des composés hydroxyliques de formule II dans lesquels R représente un radical aliphatique, araliphatique, cycloaliphatique, cycloaliphatique-aliphatique ou hétérocyclique-aliphatique qui contient au plus 120 atomes de carbone et dont la valence est égale à n, et n désigne un nombre de 2 à 6.

3. Procédé selon la revendication 1 caractérisé en ce qu'on utilise, par équivalent d'hydroxy du composé de formule II, de 0,9 à 2,5 mol de chlorure ou de bromure d'allyle.

4. Procédé selon la revendication 1 caractérisé en ce qu'on utilise, comme composés de formule II, des alcools primaires ou secondaires.

5. Procédé selon la revendication 1 caractérisé en ce qu'on effectue la réaction en présence d'une solution aqueuse de NaOH d'une concentration comprise entre 20 et 99%.

6. Procédé selon la revendication 1 caractérisé en ce qu'on effectue la réaction en présence d'une solution aqueuse de NaOH d'une concentration comprise entre 30 et 80%.

7. Procédé selon la revendication 1 caractérisé en ce qu'on effectue la réaction dans l'intervalle de température allant de 25 à 80 °C.

8. Procédé selon la revendication 1 caractérisé en ce qu'on effectue la réaction dans l'intervalle de température allant de 50 à 75 °C.

9. Ethers allyliques de formule I selon la revendication 1, caractérisés en ce que R représente un radical répondant à l'une des formules:

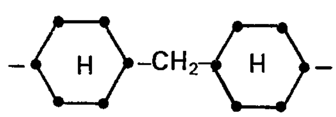

et

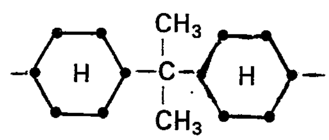

et n désigne le nombre 2.

10. Mélange d'isomères d'éthers diallyliques des o,o-, o,p- et p,p-perhydro-bis-phénols F comme composés de formule I selon la revendication 1.

## Claims

1. Process for the production of allyl ethers of the formula I

$R-(O-CH_2-CH=CH_2)_2$ (I)

wherein R is an n-valent aliphatic, araliphatic, cycloaliphatic, cycloaliphatic-aliphatic or heterocyclic-aliphatic radical, and n is a number of at least 2, by reaction of hydroxy compounds of the formula II

$R-(OH)_n$ (II)

wherein R and n are as defined in formula I, and the OH groups are bound to primary, secondary or tertiary aliphatic C atoms, with allyl chloride or allyl bromide in the presence of a catalyst and in an alkaline medium, characterised in that the reaction is performed by means of phase transfer catalysis, 0.8 to 5 mols of allyl chloride or allyl bromide, 1 to 6 mols of aqueous or solid sodium hydroxide and, as the phase transfer catalyst, 2 to 20 mol % of a quaternary ammonium salt, a quaternary ammonium base or a crown ether being employed per hydroxyl equivalent of the compound of the formula II; and the reaction is carried out in the temperature range of 20 to 100 °C.

2. Process according to claim 1, characterised in that there are used hydroxy compounds of the formula II wherein R is an n-valent aliphatic, araliphatic, cycloaliphatic, cycloaliphatic-aliphatic or heterocyclic-aliphatic radical having up to 120 C atoms, and n is a number from 2 to 6.

3. Process according to claim 1, characterised in that 0.9 to 2.5 mols of allyl chloride or allyl bromide are used per 1 hydroxyl equivalent of the compound of the formula II.

4. Process according to claim 1, characterised in that primary or secondary alcohols are used as compound of the formula II.

5. Process according to claim 1, characterised in that the reaction is performed in the presence of a 20 to 99% aqueous NaOH solution.

6. Process according to claim 1, characterised in that the reaction is performed in the presence of a 30 to 80% aqueous NaOH solution.

7. Process according to claim 1, characterised in that the reaction is performed in the temperature range of 25 to 80 °C.

8. Process according to claim 1, characterised

in that the reaction is performed in the temperature range of 50 to 75 °C.

9. Allyl ethers according to formula I of claim 1, characterised in that R is a radical of the formulae

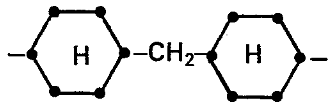

or

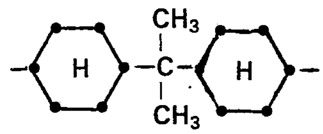

and n is the number 2.

10. Isomeric mixture of o,o-, o,p- and p,p-perhydrobisphenol F diallyl ethers as compounds of the formula I according to claim 1.